# EUROPEAN PATENT APPLICATION

(11) **EP 2 172 446 A1**
(43) Date of publication of application: **07.04.2010**
(21) Application number: 09167722.9
(22) Date of filing: 12.08.2009
(51) Int. Cl.: C07C 235/10, C08G 69/04, C08G 69/44

(54) **In situ synthesis of ester-amide-containing molecules**

(30) Priority: 13.08.2008 US 88543 P
(71) Applicant: DOW GLOBAL TECHNOLOGIES INC., Midland, MI 48674 (US)
(72) Inventor: Harris, William, J., Lake Jackson, TX 77566 (US); Broos, Rene, 2880 Bornem (BE); Jimenez, Jorge, Lake Jackson, TX 77566 (US)
(74) Representative: Raynor, John

(57) **Abstract**

A method of forming a reactive composition for directly synthesizing polyesteramides and copolyesteramides, comprises forming an initial reaction mixture comprising a lactone, an amine, and an ester as a latently-reactive diluent, and reacting the lactone and amine to form a reactive composition which is a mixture of a hydroxy-amide compound and the latently-reactive diluent ester. The composition is useful for producing self-assembling copolyesteramides, useful for manufacturing self-assembling materials.

## Description

### TECHNICAL FIELD

The invention pertains to direct methods for synthesizing hydroxy-amide-containing monomers and compounds, and their subsequent reactions.

### BACKGROUND

Hydroxy amide compounds can be useful as monomers in making oligomers or polymers such as polyesteramides and copolyesteramides. Typically, preparing such monomers for use in polymerization reactions has required numerous synthetic steps including isolation and purification of certain intermediates between reaction steps. Previously, these steps have been believed necessary to more consistently prepare materials having desirable physical properties such as improved thermal transitions, especially sharper melting point range and/or higher melting temperatures.

In particular, prior approaches to preparing these materials have focused on eliminating solvents, and/or multiple recrystallization steps. But, solvent elimination tends to result in extensive side-reactions from uncontrolled temperature spikes when reacting in neat/or high reactant concentration processes thereby reducing product yields and/or product purity, and negatively affecting molecular weight. Additionally, when products are produced in high concentrations the resulting products often require additional heat energy to initiate or maintain adequate product flow, or they can require mechanical removal from a reactor, or reaction vessel, prior to subsequent processing.

### BRIEF SUMMARY OF THE INVENTION

Surprisingly applicants have discovered a method that enables direct formation of the hydroxyamide without need for solvent elimination steps and without the drawbacks of forming the compound neat (i.e., without solvent). The method employs a latently reactive ester as a diluent and prepares a reactive composition comprising a mixture of the hydroxyamide and the latently reactive diluent ester. More surprisingly, applicants have discovered that if the reactive composition is used in a subsequent polymerization reaction of the latently reactive diluent ester and the hydroxyamide, the resulting polymer or oligomer has a higher melting point and better properties than when a latently reactive diol is used instead of the latently reactive diluent ester.

Thus, a first aspect of the invention relates to a method of forming a reactive composition, the method comprising the steps of forming an initial reaction mixture comprising a lactone, an amine, and at least one latently-reactive ester as a diluent, and reacting the lactone and amine to form a reactive composition comprising a mixture of a hydroxy-amide containing compound and the at least one latently-reactive diluent ester. The latently-reactive diluent ester does not react or exhibits only minimal reaction in the initial reaction, and does not negatively effect subsequent reactions. No substantial amount of an additional solvent is added to the initial reaction mixture. The amine(s) can have one or more amine functional groups (e.g. a monoamine (one amine functional group), diamine (two amine functional groups) or triamine (three amine functional groups)). The lactone or lactones can be added in amounts so as to yield on reaction with amine or amines a mono-hydroxy amide, dihydroxydiamide, or other multi-hydroxy functional amide, and the like. Such amide is useful as a chemical intermediate, preferably a monomer intermediate in a subsequent polymerization reaction. Preferably, the method further comprises reacting in a polymerization reaction the latently-reactive diluent ester (as a diester) with the hydroxyl amide (optionally in the presence of a diol) thereby forming an esteramide, preferably, a polyesteramide or copolyesteramide (in the event a diol is present). The copolyesteramide materials produced to the method of the invention exhibit higher melting temperatures than those of materials prepared by conventional methods, making the copolyesteramide materials useful in applications requiring higher temperatures (e.g., automotive applications).

### DETAILED DESCRIPTION

Incorporating at least one latently-reactive diluent ester into the initial reaction mixture used to form a hydroxyamide, preferably a dihydroxydiamide monomer, benefits from such reaction schemes because the need for isolating and/or purifying the subsequently formed dihydroxydiamide-containing compound is reduced or eliminated, thereby allowing the dihydroxydiamide-containing compound to be reacted *in situ* in a subsequent reaction with at least one of the at least one latently-reactive diluent ester. Without being bound by theory, the inventors believe that the latently-reactive diluent ester functions, at least in part, as a solvent for the reaction between the amine and lactone. In addition the inventors surprisingly found that the melting point of a polymer, such as a copolyesteramide, subsequently made by reacting *in situ* the dihydroxydiamide monomer with the latently reactive diluent ester is higher compared to when other solvating additives such as typical diol monomers are used. Additionally, there is better incorporation of the amine as the amide in the polymerization product in contrast to reactions with just typical solvating additives such as diol monomers. Another benefit is that reaction heat is generally more uniformly dissipated and transferred to a reaction vessel because the reaction media is diluted; hence the temperature rise is less. Finally, product processability is enhanced because the final reaction mixture can be a fluid, albeit sometimes viscous, or slurry that liquefies at lower temperatures in contrast to the typically high temperature melting crystalline product that results from the reaction sequence using neat reactants. Preferably, the initial reaction mixture consists essentially of the lactone, the amine and the latently-reactive diluent ester; wherein substantially no additional solvent is added to the initial reaction mixture. The phrase "no substantial amount of an additional solvent" means that while an incidental amount of solvent may be present it does not have significant effect on the reaction by significant change in viscosity of the mixture or by serving as a significant heat sink for the heat arising from the reaction. Preferably, the amount of additional solvent is less than 2% by weight, more preferably less than 1% by weight, and most preferably no additional solvent is added, the percent by weight being based on total weight of the initial reaction mixture.

For the purposes of the present disclosure, "heteroalkenylene" is an alkenylene group that contains one or more non-carbon atoms in an otherwise hydrocarbon group (e.g., replacing one or more non-neighboring CH₂ groups with O, S, etc.) and/or the hydrocarbon chain has one or more non-hydrocarbon substituents such as halides, alkoxy, hydroxy, thiol, or ketone groups, oxygen containing heteroalkenylene groups. A "heteroalkylene" is an alkylene group that contains one or more non-carbon atoms in the otherwise hydrocarbon chain (replacing one or more non-neighboring CH₂ groups with O, S, etc.) and/or the hydrocarbon chain has one or more non-hydrocarbon substituents such as can be optionally halides, alkoxy, hydroxy, thiol, ketone groups. Heteroalkylene groups containing oxygen are preferred. "Heteroarylene" is an arylene group that contains one or more non-carbon atoms in the aromatic group (e.g., replacing one or more non-neighboring CH groups with O, S, N etc.) and/or one or more aromatic carbons has one or more non-hydrocarbon substituents such as halides, alkoxy, hydroxy, thiol, ketone, ester, or acid groups. Hydrocarbylene groups are divalent hydrocarbon moieities. Heterohydrocarbylene groups are groups having one or more non-carbon atoms in the otherwise hydrocarbylene moiety (e.g. replacing one or more non-neighboring CH₂ groups with O, S, etc.) and/or having halides, alkoxy, hydroxy, thiol, ketone, acid or ester substituent groups.

Accordingly, in a first aspect, the invention relates to a method comprising the steps of forming an initial reaction mixture comprising a lactone, an amine, and a latently-reactive diluent ester, and reacting the lactone and amine to form a hydroxyamide-containing compound while the latently-reactive diluent ester does not react or exhibits only minimal reaction, and does not negatively effect subsequent reactions. The lactone comprises at least one lactone functional group (i.e., -C^{a}(=O)-O-C^{b}- where the -C^{a}-O-C^{b}- are atoms comprising a ring of the lactone). The lactone can comprise an alkylene, alkenylene, heteroalkylene, or heteroalkenylene group comprising 3 to 24 carbon atoms, each group comprising the C^{b}. Alkylene groups are most preferred. Preferably, the lactone can comprise 3 to 18 carbon atoms, and more preferably, 4 to 12 carbon atoms with alkylene and alkenylene groups being preferred. Lactones comprising 3, 4, 5, or 6 carbon atoms in the ring are most preferred and can be substituted or unsubstituted β-propiolactone, γ-butyrolactone, δ-valerolactone, ε-caprolactone, pentadecalactone, glycolide, and lactides. ε -Caprolactone is especially preferred. The lactone can be substituted with at least one alkyl-, alkenyl-, aryl-, heteroalkyl-, heteroalkenyl-, or heteroaryl- group: the definitions for these univalent groups corresponding to the divalent groups listed above.

The amines useful in the present invention include amine containing compounds preferably comprising at least two amine functional groups (e.g. R(NH₂)₂). If the diamine is a monomer, primary amines are preferred (i.e. -NH₂ groups), but the amine functional group may also be secondary amines. The R groups can be hydrocarbylene or heterohydrocarbylene and include: linear, branched, or cyclic-containing alkenylene, alkylene, heteroalkylene, or heteroalkenylene groups having 2 to 24 carbon atoms, and arylene, or heteroarylene groups having 5 to 24 carbon atoms. In a preferred embodiment, the amine can be a primary and/or secondary diamine: primary diamines are more preferred. In another preferred embodiment, the diamine is primary alkenylene or alkylene diamine, and in a most preferred embodiment, the diamine is linear alkenylene or alkylene-containing diamine. Mixtures of amines can be used, but it is preferred to use one amine-containing compound. Exemplary non-limiting diamine embodiments include ethylene diamine, 1,4-diaminobutane, 1,6-diaminohexane, 1,3-diaminopropane, 1,12-diaminododecane, 1,3-diaminopentane, 2-methylpentamethylenediamine, cyclohexanedimethanamine; and their isomers. Especially preferred diamine embodiments include ethylene diamine, 1, 4-diaminobutane, and 1, 6-diaminohexane.

The initial reaction mixture further comprises a latently reactive diluent ester which serves as a diluting agent in the initial reaction step of forming hydroxy-amide containing compounds and preferably is subsequently reacted with the hydroxy-amide compound to form esteramides, more preferably polyesteramides, and copolyesteramides. A diluent ester compound can have one or more ester functional groups (i.e., -C(=O)-O-C): preferred are diluent esters having at least two ester functional groups ("diester") are useful as monomers. The diluent ester compound can comprise a hydrocarbylene or heterohydrocarbylene group having 2 to 24 carbon atoms including linear, branched, or cyclic group-containing alkenylene, alkylene, heteroalkylene, or heteroalkenylene group, arylene or heteroarylene, or a dialkyloxalate (e.g. oxalic acid esters) group having 2 to 24 carbon atoms. Non-limiting examples of latently reactive diester compounds according to the above definitions include: dimethyl- or diethyl- succinate, dimethyl adipate, dimethyl oxalate, dimethyl malonate, dimethyl glutarate, dimethyl azelate, dimethyl pimelate, dimethyl dodecanoate, dimethyl sebacate, and dimethyl suberate. Preferred latently reactive ester compounds include dimethyl adipate, dimethyl sebacate, and dimethyl azelate, and dimethyl dodecanoate. An especially preferred latently reactive ester compound is dimethyl adipate.

### Hydroxyamide in-situ synthesis

Lactone and diamine compounds are reacted together, in the absence of a catalyst, but in the presence of the latently-reactive diluent ester, where the ester does not substantially participate in the reaction, to form hydroxy-amide compounds, preferably dihydroxydiamide compounds. The dihydroxydiamide compounds are useful as monomers in the synthesis of molecularly self-assembling molecules. The lactone and diamine compounds are preferably present in approximately stoichiometric amounts (e.g. about 1 mole diamine for every 2 moles lactone). Depending on the specific nature of the lactone and diamine used, useful reaction temperature is greater than about 0°C, preferably greater than about 50°C more preferably greater than about 75°C. Reaction temperature is less than 225°C, preferably less than 190°C and more preferably less than 160°C. It is desirable to minimize the overall temperature history (the length of time and degree of heating) needed to form hydroxyl-amide compounds to that necessary to drive the reaction of amine with lactone to high conversions yielding the hydroxy-amide. The reaction can be conducted at a constant temperature, a variable temperature, or over a series of temperatures, by varying the temperature in a constant fashion or in a step-wise fashion. Additionally, the reaction to form hydroxy-amide compounds can be conducted at a constant or variable pressure. Reaction pressures can be subatmospheric, atmospheric, and/or super-atmospheric, preferably from 3040 torr to about 0.001 torr, more preferably from 2280 torr to about 0.01 torr, and still more preferably from about 760 torr to about 0.1 torr although other pressures and combinations of pressures can be used. It is desired that the combination of temperature and pressure minimize the loss of lactone and amine until their reaction is mostly completed (as would be practiced in the art). Preferably the reaction should be more than 90% completed, more preferably more than 95% completed. In certain embodiments of the invention, the method is conducted under an inert atmosphere such as that provided by nitrogen or argon blanket, or other gaseous mixtures that can prevent, exclude, or reduce unwanted reactions, for example unwanted oxidation. The reaction can be performed step-wise, continuously, or a combination of steps thereof at various combinations of time, temperatures and pressures so as to yield the desired product and minimize to the degree possible any formation of undesired side reactions. The method can further comprise the step of reacting the lactone and amine in one or more reaction zones. In certain other preferred embodiments, the hydroxyamide and the latently reactive ester diluent solution are fluid at ambient conditions, or fluid at a temperature that is lower than the melting point of the pure hydroxyamide.

The quantity of latently reactive diluent ester that can be added to the reaction mixture is flexible, depending on several1) the desired subsequent product (preferably, for example, a copolyesteramide) and/or 2) the degree of thermal/mass transfer desired to allow the hydroxyamide in-situ_reaction to proceed to high yield/conversion, preferably with little or no loss of reactants. The diluent ester is added in amounts preferably less than about 95 weight percent, more preferably, less than about 75 weight percent, still more preferably less than about 50 weight percent, and most preferably less than about 25 weight percent based of the total mass of the initial reaction mixture. In one preferred embodiment, the amount of latently reactive ester diluent used in the preparation of the dihydroxydiamide is preferably at least one half mole, more preferably at least one mole, of ester diluent per 1 mole diamine and 2 moles of lactone to prepare a dihydroxydiamide.

When the diluent ester is also used in subsequent reactions, where preferably a polymeric product is formed, preferably a polyesteramide or copolyesteramide, the latently reactive diluent ester (in the embodiment of a diester) is added in amounts that allow the subsequent polymerization reaction(s) to run to high conversion of reactants, high molecular weight, and high amide group incorporation into the polymer maximized.

### Polyesteramide synthesis

The hydroxy-amide (particularly dihydroxydiamide) can be reacted with the latently reactive diluent ester to form compounds comprising an esteramide, preferably a polyesteramide, and a most preferably a copolyesteramide. The polyesteramide is formed by reacting a latently reactive diluent diester with a dihydroxydiamide, preferably using a catalyst and/or heating and vacuum preferably using reaction conditions known in the art. The copolyesteramide can be formed by adding at least one additional diol-, optionally including a higher functional hydroxy-functional compound (e.g. tri-hydroxy-functional containing compound) to the reactive composition comprising a mixture of a dihydroxydiamide and a latently reactive diluent diester, preferably in the presence of a catalyst, preferably with heating and vacuum. In the polyesteramide and copolyesteramide preparation, additional diester compounds, and/or/diol compounds, can be added to form various oligomeric or polymeric structures. The diol is preferably a linear, branched or cyclic aliphatic or hetereoalkylene diol having from 2- 24 carbons. The diol can also be a polyol with a number average molecular weight from about 100 to about 5000. Useful preferred diols, and polyols, include: 1,4-butanediol, 1,2-ethanediol, 1,3-propanediol, 1,6-hexanediol, cyclohexanedimethanol, neopentyl glycol, polytetramethylene glycol, polypropylene glycol, polyethylene glycol, polyalkylene glycol, polyalkenylene glycol and the like: varying molecular weights of these compounds are well known in the art. The most preferred diols include 1,4-butanediol, 1,2-ethanediol, and 1,6-hexanediol.

In one embodiment, the polyesteramide is a self assembling polyesteramide, or a self assembling copolyesteramide. The polymerization reaction is preferably performed in sequence subsequent to the hydroxy-amide synthesis by adding one or more esterification/ transesterification catalyst, or catalysts, to the hydroxyamide and latently reactive diluent ester mixture. One class of preferred catalysts includes titanates, most preferably titanium (IV) butoxide (Ti(OBu)₄). Useful catalysts include: aluminum (III)isopropoxide (Al[OCH(CH₃)₂)]₃), antimony oxide (Sb₂O₃), antimony acetate, dibutytin oxide, tin octoate and other esterification catalysts.

Various processes known that can be useful for preparing polyesteramides and copolyesteramides (and are quite similar to those processes known to prepare polyesters) can be used to prepare self-assembling polyesteramide and copolyesteramide polymers using the hydroxyamides of the present invention. Generally, polyesteramides and copolyesteramides are prepared by melt polymerization. For copolyesteramides, preferably there is at least one diester for every diol monomer, and preferably there is a stoichiometric excess of diol monomer to diester at the start of the polymerization process, preferably excess diol monomer can be distilled off from the reaction mixture during the polymerization reaction to increase the product molecular weight to a desired value; the technique is known in the art. The polymerization is carried out in the presence of one of the many known polyester catalysts, preferably titanium (IV) butoxide, and preferably at elevated temperature: the range can be from about room temperature, preferably above 40°C, more preferably above 80°C, and still more preferably above 130°C. The reaction can be performed at 300°C or less, preferably 250°C or less, more preferably 200°C or less, and still more preferably 180°C or less; a preferred range for the reaction temperature is from about 165°C to about 300°C with pressure reduction as needed to facilitate molecular weight increase (to remove alcohols) as is known in the polymerization art. The reaction can be continued until desired quantities of polymer product having a desired molecular weight, or molecular weight range, are formed. In a typical embodiment, the reaction can be run over a time range of from about 0.1 to 60 hours, preferably from about 0.1 to 30 hours, most preferably from about 0.1-24 hours. The pressure in the reaction may be reduced to facilitate removal of volatile components such as alcohols or excess diols or other additive. The pressure reduction can be over a range to volatilize higher molecular weight alcohols or excess diols and is not particularly restricted. The pressure reduction can be down to about 1 Torr if needed, preferably down to about 0.1 Torr, more preferably down to about 0.001 Torr.

The temperature of the reaction can subsequently be lowered to room temperature, or a useful product handling temperature, and the product polymer removed from the reaction vessel, or the polymer can be removed from the reactor while molten with the temperature elevated as needed. In certain embodiments it is possible to control the polyesteramide or copolyesteramide molecular weight by "off-stoichiometry" (i.e. not exactly equal numbers of functional groups to react between different monomer types) combinations of the monomers, and/ or using a terminating agent such as a monoacid, monoester, mono-alcohol, monoamine, and/or other monofunctional reactive species, or combination thereof useful in preparing the polymer product as is known in the art: these materials can preferably be added at any point during the polymerization so as to control the desired properties of molecular weight and/or distribution. A branched polymeric material can be prepared by adding a reactive trifunctional monomer species, or even higher polyfunctional species, at some point in the polymerization process. The addition of the polyfunctional monomer can be used to prepare a self-assembling polyesteramide, or copolyesteramide.

The monomer ratios can be varied over a large combination of molar ratios of from 20 moles of diesters to one diamine, to one mole of diester to one diamine. The range is preferably a mole ratio of 15 moles diesters to one diamine, more preferably a mole ratio of 10 moles diesters to one diamine, even more preferably a mole ratio of 5 moles diesters to 1 diamine, and most preferably, a mole ratio of two moles of latently reactive diester diluent can be initially added for each initial mole of diamine. A copolyesteramide can be prepared if the mole ratio of diester to diamine is greater than 1, with the addition of a diol. For a copolyesteramide, the minimum amount of total diol moles (e.g. added diol + dihydroxydiamide) is equal to the total moles of diluent diester, preferably 1.5 moles or more total diols per mole of diluent ester, more preferably 2.0 or more moles total diols per mole of diluent ester, and most preferably 5 or more moles of total diol per mole of diluent ester. The amount of total diols per mole of diluent ester is less than 10 moles of diols per mole of diluent ester, and more preferably less than 7.5 moles of diols per mole of diluent ester. To produce different copolyesteramides, more than one different additional diol can be added, as can more than one latently reactive diester diluent- the amount and quantity of each determining the final micro- repeating structure of the copolyesteramide. In a preferred embodiment, the desired copolyesteramide is a self-assembling copolyesteramide.

It is also possible to add stabilizers, processing aides, fillers, colorants during the process of preparing monomers, polymers, or after their formation as would be known in the art.

Embodiments made by any one of the preceding methods (to produce a polyesteramide or copolyesteramide) can have an Mₙ of at least about 2000 g/mole, preferably greater than 3000 g/mol, more preferably greater than 4000 g/mol, still more preferably greater than 7000 g/mol, even still more preferably greater than 12,000 g/mol, and most preferably greater than 18,000 g/mol. In other embodiments, the number average molecular weight can be less than about 50,000 g/mol, more preferably less than 30,000 g/mol, most preferably less than 25,000 g/mol. A preferred molecular weight range is from about 3,000 to about 40,000 g/mol wherein the average number molecular weight (Mₙ) as determined by proton NMR, gel permeation chromatography, or other technique known in the polymer art.

Preferably, the polymer formed is a self assembling material. The term "self-assembling material" means an oligomer or polymer that effectively behaves larger associated or assembled oligomers and/ or polymers through the physical intermolecular associations of chemical functional groups in certain states while still being processable according to processes consistent with its molecular weight.

### EXAMPLES

### Polymer characterization

Proton NMR are performed preferably on a Bruker 250 MHz spectrometer on typical 1-10 weight percent solutions typically in a solvent of d4-acetic acid as is known in the art. Proton NMR can be used to determine copolymer repeat unit composition, and copolymer number average molecular weight, preferably by utilizing the number of CH₂OH endgroups in calculating the molecular weight: such procedures are known in the polymer arts. Proton NMR assignments are dependent on the specific structure being analyzed as well as the solvent, concentration, and temperatures used for measurement. For copolyesteramides, d4-acetic acid is preferably as a convenient NMR solvent. The molecular weight method can involve comparing the resonances of various groups in the polymers and calculating ratios of, for example, end groups -(CH₂OH) to various other groups that are contained in the other repeat units.

Differential scanning calorimetry (DSC) can be done with a TA Instruments 2920 or Q100 or other similar thermal analysis instrument that is known in the polymer arts: typically heating rates and cooling rates of 10°C/min are employed with the DSC technique as is known in the art to determine thermal transitions characteristic of the material. Melting points are taken as the endothermic maxima in a melting transition upon the second DSC scan; the second scan is known in the art to be more typically a measurement of the native thermal polymer property without previous material processing history. Inherent viscosities can be measured with ∼0.5 g/dL(grams per deciliter) solutions at 30°C in an appropriate solvent such as chloroform/methanol (1/1), or other known polymer solvent; viscosity testing is a useful known polymer property. Tensile testing, if needed, can be performed according to ASTM 1708 using microtensile specimen geometry on an Instron 5581 to determine mechanical properties such as yield stress, modulus and the like. Such properties are well known in the polymer mechanical property art.

### Example 1. Production of an MSA copolyesteramide using in-situ hydroxy-amide formation and sequential addition of a diol monomer.

Ethylenediamine (6.03 g, 0.100 mole), dimethyl adipate (34.94 g, 0.2006 mole), and ε-caprolactone (22.88 g. 0.2004 mole) are loaded into a 2-neck, 250 mL round bottom flask and placed under a nitrogen atmosphere, The flask is stirred using an overhead glass stir-shaft and blade equipped with bearing/seal overnight: no heat is applied and the mixture is stirred overnight. A nitrogen-inlet distillation head with Vigreux column is wrapped with heating tape and insulated and a take-off adaptor and receiver are inserted into the flask whereupon the flask is immersed into a 160°C constant temperature bath for 2 hours). The flask is subsequently cooled to room temperature. 1,4-Butanediol (26.6 mL, 0.300 mole) and titanium (IV) butoxide (0.106 mL, 0.31 mmol) are injected into the flask whereupon it is immersed in the 160°C bath with temperature set point immediately being raised to 175°C and heated for a total of 2 hours, the flask heating up to the bath set temperature 175°C at atmospheric pressure. The pressure in the apparatus is reduced and the reaction run according to the following timed sequence schedule: 5 minutes at 100 Torr, then 5 minutes at 50 Torr, then 5 minutes at 40 Torr, then 10 minutes at 30 Torr, then 10 minutes at 20 Torr, then 60 minutes at 10 Torr, then 60 minutes at approximately 0.6 Torr at 175°C, then 60 minutes at about 0.5 Torr and the bath setpoint is changed to 190°C; the ; the setpoint is raised to 210°C and the flask is heated for 180 minutes at about 0.4 Torr. The polymer product is recovered and is found to have the following physical properties: inherent viscosity is 0.37 dL/g (deciliters per gram (methanol/chloroform)equal weight ratios: one to one w/w), viscosity measurement is taken at 30.0°C, using a 0.50 g/dL solution. The number average molecular weight, Mₙ is 11,400 and a 48 mole % diamide content via proton NMR (with the sample dissolved in d4-acetic acid) from the condensation of ethylene diamine and ε-caprolactone is found. The DSC thermal transitions are determined at a heating rate of 10°C/min with 77°C and 121°C melting points. Tensile bars are prepared and the mechanical properties are determined via ASTM D1708. The microtensile data are an average of the values for 5 specimens: Strain at break is 813±250 percent. The modulus (run in the instrument's "Automode") is 188±12 MPa. The modulus (tangent at 0.15% elongation) is 86±21 MPa. The stress at maximum Load is 19.5±5.3 MPa. The energy at sample break is 32.5±15.3 in-pounds.

### Example 2. Production of a large batch copolyesteramide via in-situ production of di-hydroxy-di-amide in presence of a diluent ester and subsequent polymerization of di-hydroxy-di-amide, diluent ester and a diol.

The reaction of Example 1 was repeated on a larger scale using same reaction assembly as Example 1, except with appropriately larger containers: a 500 mL roundbottom flask was charged first with ethylene diamine (21.11g, 0.351 mole), dimethyl adipate (122.29 g. 0.702 mole), ε-caprolactone (80.08 g, 0.702 mole), followed later with a second charge of 1, 4-butanediol (93.1 mL, and 1.06 mole), and titanium (IV) butoxide The heating and vacuum sequence used in Example 1 were followed closely. A copolyesteramide product is recovered and found to have the following physical properties: inherent viscosity = 0.25 L/g (in methanol/chloroform(1/1, weight/weight), 30.0°C, 0.50 grams/deciliter concentration). Mₙ = 6750 and 49 mole% diamide incorporation is found via proton NMR (with the sample dissolved in d4-acetic acid) from the condensation of the initial ethylene diamine and ε-caprolactone. The DSC melting points are measured at 10°C/min heating rate and are 75°C and 119°C. Tensile bars were prepared and the mechanical properties were determined via ASTM D1708. Strain at break = 391%; Modulus = 138 MPascals.

### Comparative Example 1 Production of hydroxy-amide in the presence of a diluent diol and subsequent polymerization of the hydroxy-amide, diluent diol and diester to produce a copolyesteramide.

Ethylenediamine (6.03 g, 0.100 mole), 1,4-butanediol (27.09 g, 0.3006 mole), and ε-caprolactone (22.88 g. 0.2004 mole) are loaded into a 2-neck, 250 mL round bottom flask and placed under a nitrogen atmosphere, The flask is stirred using an overhead glass stir-shaft and blade equipped with bearing/seal overnight: no heat is applied with mixture stirred overnight. A nitrogen padded distillation head with Vigreux column wrapped with heating tape and insulated along with a take-off adaptor and receiver are inserted into the flask whereupon the flask is immersed into a 160°C constant temperature bath for 2 hours. The flask is subsequently cooled to about room temperature. Dimethyl adipate (33 mL, 0.320 mole) and titanium (IV) butoxide (0.106 mL, 0.31 mmol) are injected into the flask whereupon it is immersed in the 160°C bath with temperature set point immediately being raised to 175°C for total of 2 hours heating up to and at the bath set temperature 175°C. Reduced pressure is applied to the apparatus according to the following timed schedule: 5 minutes at 100 Torr, then 5 minutes at 50 Torr, then 5 minutes at 40 Torr, then 10 minutes at 30 Torr, then 10 minutes at 20 Torr, then 60 minutes at 10 Torr, then 60 minutes at approximately 0.6 Torr at 175°C, then 60 minutes at about 0.5 Torr and the bath setpoint is changed to 190°C; the flask is heated to and stays at the 190 °C for 60 minutes, the setpoint is changed and the flask is heated for 180 minutes at∼0.3 Torr up to and at 210°C. The product is recovered and is found to have the following physical properties: inherent viscosity = 0.21 deciliters/grams (methanol/chloroform (1/1, weight/weight), 30.0°C, 0.50 grams/deciliters). An acceptable Mₙ = 6300 but lower 39 mole% diamide content (via proton NMR with the sample dissolved in d4-acetic acid) results from the incorporation of the condensation dihydroxydiamide from the ethylene diamine and ε-caprolactone. The resulting DSC melting points at 10°C/min heating rate are depressed to 67°C and 102°C with the melting point of the higher transition being substantially depressed using this process.

### Comparative Example 2 Production of hydroxy-amide in presence of diluent diol and diester with subsequent polymerization of hydroxy-amide, diluent diol and diester to produce a copolyesteramide.

Ethylenediamine (6.03 g, 0.100 mole), 1,4-butanediol (27.09 g, 0.3006 mole), dimethyl adipate (34.9, 0.200 mole) and ε-caprolactone (22.88 g. 0.2004 mole) are loaded into a 2-neck, 250 mL round bottom flask and placed under a nitrogen atmosphere. The flask is stirred using an overhead glass stir-shaft and blade equipped with bearing/seal overnight: no heat is applied with mixture stirred overnight. A nitrogen padded distillation head with Vigreux column wrapped with heating tape and insulated along with a take-off adaptor and receiver are inserted into the flask whereupon the flask is immersed into a 160°C constant temperature bath for 2 hours. The flask is subsequently cooled to about room temperature and titanium (IV) butoxide (0.106 mL, 0.31 mmol) is injected into the flask whereupon it is immersed in the 160°C bath with temperature set point immediately being raised to 175°C for total of 2.2 hours heating up to and at the bath set temperature 175°C. Reduced pressure is applied to the apparatus according to the following timed schedule: 5 minutes at 100 Torr, then 5 minutes at 50 Torr, then 5 minutes at 40 Torr, then 10 minutes at 30 Torr, then 10 minutes at 20 Torr, then 60 minutes at 10 Torr, then 60 minutes at approximately 0.7 Torr at 175°C, then 60 minutes at about 0.5 Torr and the bath setpoint is changed to 190°C; the flask is heated to and stays at 190 °C for 60 minutes, the setpoint is changed and the flask is heated for 180 minutes at∼0.3 Torr up to and at 210°C. The product is recovered and is found to have the following physical properties: inherent viscosity is 0.26 deciliters/gram (methanol/chloroform (1/1, w/w), 30.0°C, 0.50 gram/deciliter). Mₙ = 7600 and 45 mole% C2C amide content via proton NMR (with the sample dissolved in d4-acetic acid.) The DSC melting points at 10°C/min heating rate are 85°C and 112°C with the melting point of the higher transition being substantially depressed in using this process.

### Comparative Example 3. Production of hydroxy-amide in presence of diluent ester and monoalcohol and polymerization of hydroxy-amide, diluent ester and diol to produce a copolyesteramide.

Ethylenediamine (6.03 g, 0.100 mole), dimethyl adipate (34.94 g, 0.2006 mole), anhydrous methanol (35 mL) and ε-caprolactone (22.88 g. 0.2004 mole) are loaded into a 2-neck, 250 mL round bottom flask and placed under a nitrogen atmosphere, The flask is stirred using an overhead glass stir-shaft and blade equipped with bearing/seal overnight: no heat is applied with mixture stirred overnight. A nitrogen padded distillation head with Vigreux column wrapped with heating tape and insulated along with a take-off adaptor and receiver are inserted into the flask whereupon the flask is immersed into a 160°C constant temperature bath for about 2 hours. The flask is subsequently cooled to about room temperature. 1,4-Butanediol (26.6 mL, 0.300 mole) and titanium (IV) butoxide (0.106 mL, 0.31 mmol) are injected into the flask whereupon it is immersed in the 160°C bath with temperature set point immediately being raised to 175°C for total of 2 hours heating up to and at the bath set temperature 175°C. Reduced pressure is applied to the apparatus according to the following timed schedule: 5 minutes at 100 Torr, then 5 minutes at 50 Torr, then 5 minutes at 40 Torr, then 10 minutes at 30 Torr, then 10 minutes at 20 Torr, then 60 minutes at 10 Torr, then 60 minutes at approximately 0.6 Torr at 175°C, then 60 minutes at about 0.5 Torr and the bath set point is changed to 190°C; the flask is heated to and stays at the 190°C for 60 minutes, the setpoint is changed and the flask is heated for 180 minutes at ∼0.4 Torr up to and at 210°C. The product is recovered and is found to have the following physical properties: inherent viscosity = 0.30 deciliter/gram (methanol/chloroform(1/1, w/w), 30.0°C, 0.50 grams/deciliter). Mₙ = 7530 and 47 mole% C2C amide content via proton NMR (with the sample dissolved in d4-acetic acid.). The DSC melting point at a 10°C/min heating rate is 94°C with the melting point being substantially depressed in using this process.

While the invention has been described above according to its preferred embodiments, it can be modified within the spirit and scope of this disclosure. This application is therefore intended to cover any variations, uses, or adaptations of the instant invention using the general principles disclosed herein. Further, the instant application is intended to cover such departures from the present disclosure as come within the known or customary practice in the art to which this invention pertains and which fall within the limits of the following claims.

## Claims

1. A method of forming a reactive composition, the method comprising: forming an initial reaction mixture comprising a lactone, an amine, and at least one latently-reactive diluent ester, the lactone comprising at least one lactone functional group, the amine comprising at least one amine functional group, and each of the at least one latently-reactive diluent ester independently comprising at least one ester functional group; and reacting the lactone and amine to form a reactive composition comprising a mixture of a hydroxy-amide compound and the at least one latently-reactive diluent ester.

2. The method of claim 1 wherein the initial reaction mixture consists essentially of the lactone, the amine, and the at least one latently-reactive diluent ester.

3. The method of claim 1 wherein substantially no additional solvent is added to the initial reaction mixture.

4. The method according to any of claims 1 to 3 wherein the amine has at least two primary amine functional groups and the the hydroxy-amide containing compound is a dihydroxydiamide.

5. The method according to any one of the preceding claims wherein the latently-reactive diluent ester has at least two ester functional groups.

6. The method of claim 5 further comprising reacting the dihydroxydiamide with the latently-reactive diluent ester in a polymerization reaction to form a polyesteramide.

7. The method of claim 5 further comprising adding a diol, and reacting the dihydroxydiamide, the latently-reactive diluent ester, and the diol in a polymerization reaction to form a copolyesteramide.

8. The method of claim 6 or 7 wherein the polyesteramide or copolyesteramide is a self assembling material.

9. The method according to any one of the previous claims wherein the lactone and the amine are reacted at from 0°C to 225°C.

10. The method according to one of claims 6 to 8 wherein the polymerization reaction is conducted from about 0°C to about 300°C.

11. The method according to anyone of the preceding claims wherein the mole ratio of the amine functional groups to the lactone functional groups is from 1:1 to 1:2.5.

12. The method of any one of the preceding claims wherein the mole ratio of the latently reactive diluent ester to the amine is from 20:1 1 to 1:1.

13. The method of claim 7 wherein the mole ratio of total latently-reactive diluent ester comprising at least two ester functional groups to total diol is from 1:1 to 1:10.

14. The method of claim any one of the preceding claims wherein the lactone is β-propiolactone, γ-butyrolactone, δ-valerolactone, ε-caprolactone, pentadecalactone, or glycolide; the amine is ethylene diamine, 1, 4-diaminobutane, or 1,6-diaminohexane; and the diol is 1,4-butanediol, 1,2-ethanediol, or 1,6-hexanediol.

15. The method of claim any one of the claims 6 to 8 wherein the polyesteramide or copolyesteramide has a molecular weight of from 3,000 grams per mole to 40,000 grams per mole.
